# EUROPEAN PATENT APPLICATION

(11) **EP 1 405 616 A1**
(43) Date of publication of application: **07.04.2004**
(21) Application number: 03022570.0
(22) Date of filing: 02.10.2003
(51) Int. Cl.: A61F 2/44

(54) **Method for correcting a deformity in the spinal column and its corresponding implant**

(30) Priority: 02.10.2002 AR 0203711
(71) Applicant: Carrasco, Mauricio Rodolfo, City of Buenos Aires (AR)
(72) Inventor: Carrasco, Mauricio Rodolfo, City of Buenos Aires (AR)
(74) Representative: Grünecker, Kinkeldey, Stockmair & Schwanhäusser Anwaltssozietät

(57) **Abstract**

This invention refers to an implant to be inserted in the disc space between two adjacent vertebrae for the correction of the vertebral spine curvature characterized in that its configuration (lateral view) is basically a wedge or acute-angled isosceles trapeze, wherein the area (9) opposite the shorter base or opposite to the vertex is a rounded pyramid-like surface, and the upper and lower surfaces of the trapeze include fixation means (1,2,3) to the vertebral plates of the adjacent vertebrae.

## Description

This invention refers to a method for correcting a deformity in the spinal column comprising the steps of resecting part of the intervertebral disc with its ring, resecting the joints joining said adjacent vertebrae, resecting at least part of the spinal apophysis of said adjacent vertebrae, providing an implant to be inserted between said adjacent vertebrae, inducing the separation of only the vertebral edges of the anterior part of said vertebrae; wherein by means of other devices adaptable to the external parts of the spinal column, the vertebral plates are compressed against the supporting faces of the implant, wherein the vertebral plates, when standing on the supporting faces of said implant, form an open angle forwards, which vertex is adjacent to the union of the posterior vertebral edges, where said angle is bigger than the previously existing one. As a result of the interaction of the spinal implant located between the vertebrae and the compression apparatus, a predetermined correction of a deformity of the spinal vertebrae is obtained thus enabling the final fixation of the vertebrae with the use of implant material.

### Background of the invention

### a) Technique field

This disclosure in general relates to surgical methods and implants used in the stabilization of the lumbar column for the treatment of intervertebral disc degenerations and for the correction of the thoracolumbar kyphotic deformity.

### Prior art description

### i) Surgical Techniques for Kyphotic Deformity and Lumbar Lordosis Correction.

The expanded disease of the intervertebral discs in the lumbar column region causes a reduction in the normal curvature known as lordosis. Likewise, the need of stabilizing the spinal curvature may result from an operation on said region, specially when metal assemblies have been used without correcting the defect. Numerous cases result in an inversion of the spinal curvature thus producing a kyphosis. In these circumstances, it is necessary to correct the deformity in the spinal column in order to avoid the painful consequences and physical disability.

As references to the prior art related to surgical techniques for the correction of the spinal curvature, we may mention the collection work by J. Van Royen and A. De Gast (*Lumbar Osteotomy For Correction of Thoracolumbar Kyphotic Deformity in Ankylosing Spondylitis*' and *A Structured Review of Three Methods of Treatment* by Ann Rheum Dis 1999; **58**:399-406).

J. Van Royen and A. De Gast carried out a search in literature in 41 articles published between 1945 and 1998.

Three operative techniques have been described to correct thoracolumbar kyphotic deformity at the level of the lumbar spine: (i) opening wedge osteotomy, (ii) polysegmental wedge osteotomies, and (iii) closing wedge osteotomy.

The average correction achieved with each surgical technique ranged from 37 to 40 degrees. Loss of correction was mainly reported in patients treated by open wedge osteotomy and polysegmental wedge osteotomies. Neurological complications were reported in the three techniques. Moreover, Lumbar osteotomy for correction of TLKD is a major surgery.

The aim of a spinal osteotomy is to restore both the patient's balance and the ability to see ahead to the horizon. In addition, the intervention aims to relieve compression of the abdominal viscera caused by the chest cavity and the deformity, and improves diaphragmatic respiration as well. The spinal deformity in need of curvature corrections over 10° is mainly a combination of deformities such as a thoracic hyperkyphosis and stabilization of the lumbar lordosis. The kyphotic thoracolumbar deformity is best corrected by means of an osteotomy to improve the lordosis in the lumbar spine region since a correction in the thoracic region correction is limited by the ankylosis of the costovertebral joints. Furthermore, the overall correction is greatest when the intervention is performed at the lowest possible level of the lumbar spine.

The opening wedge osteotomy technique involves two and three level osteotomies through the articular processes of L1, L2, and L3. Correction of the kyphotic deformity was achieved by forceful manual extension of the lumbar spine in an attempt to close the posterior wedge osteotomies. This manipulation caused disruption of the anterior longitudinal ligament creating an anterior monosegmental intervertebral opening wedge with elongation ofthe anterior column.

Likewsise, a two-stage anterior opening wedge osteotomy for correction of kyphotic thoracolumbar deformities is also disclosed. The proffesional first removed the laminaofL2 under local anaesthesia, followed two weeks later by an anterior release and resection of the intervertebral disc between L2 and L3. The anterior osteotomy was then wedged open and grafted with a bone block. Numerous modifications of this anterior opening wedge osteotomy have been described. The sharp lordotic angle and elongation of the anterior column occurring in this procedure were assumed to be associated with serious vascular and neurological complications. To avoid such complications, polysegmental posterior wedge osteotomies and closing wedge posterior osteotomies of the lumbar spine were introduced.

The polysegmental lumbar posterior wedge osteotomy technique achieves correction by multiple closing wedges of posterior lumbar osteotomies, including the interlaminar space and the original inferior and superior articular processes. This method gives a more gradual correction without rupturing the anterior longitudinal ligament. Posterior polysegmental lumbar wedge osteotomies, later added the use of internal fixation such as Harrington's rods and laminar hooks, and later transpedicular screws.

In the monosegmental intravertebral closing wedge posterior osteotomy technique, the posterior elements of one vertebra, including the lamina, articular processes, pedicles, in combination with the posterior wedge of the vertebral body are resected. Correction is achieved by passive extension of the lumbar spine, thus closing the posterior osteotomy, which is also favoured by the formation of an anterior hinge. An internal fixation with wiring, metal plates or transpedicular fixation has been used to ensure immediate stability and rapid consolidation.

The close wedge osteotomy is only performed in one level of the lumbar region for it is a technique that causes a division of the vertebral body into two parts. The bone wedge removed from the posterior zone is made by surgical tools and the little bone union remaining in the anterior zone of the vertebral body, more precisely in front of the bone wedge, it is abruptly broken or fractured when the osteotomy is closed by its posterior zone. In this way, a fracture line comprising all the perimeter of the vertebral body is produced.

In the article by Denis F. "The Three Column Spine and its Significance in the Classification of Acute Thoracolumbar Spinal Injuries" Spine 1983; 8:817-831, the author classifies the spine traumatic injuries in four instability grades, for which the injured vertebral anatomic part is considered and four categories are found. The fourth category being the most unstable of all refers to the injury comprising the whole vertebral body and all the bone elements behind the vertebral body. Consequently, according to their characteristics, the closing wedge osteotomy (CWO) and the anterior closing osteotomy (ACO) (Prior art 1) would be within the scope of surgical proceedings for the total spine disruption, such as the spine fracture in the fourth grade of Dennis' classification.

It becomes necessary to mechanically stabilize said mechanical instability until the fracture is healed by means of the bone bonding. In this way, two osteotomies are performed and the mechanical stabilization difficulties of the region are increased, there being a high risk of displacement of the fragments for having caused instability in two levels. Furthermore, there is also the risk that only one of the osteotomies is fixed while the other one remains unfixed.

Moreover, said surgical technique is very aggresive there being a considerable blood loss as a consequence of the fracture that has been made, the vertebral bone is removed and the muscles fixed in that part of the vertebra are detached. It is also necessary that the patient be in a very good condition to try said technique since a considerable blood loss continues after the operation.

The spine is shortened for the osteotomies are by substraction, that is to say that a posterior base osteotomy is removed. If the osteotomy were performed in two vertebrae of the same region, the shortening of the spine would be equal to the lenght of the base of both wedges, thus considerably increasing the morbidity of the proceeding and surgical risks as well.

There exist no publications where the technique in two levels of a same spine region has been attempted.

Articles "Transpedicular Decancellation Closed Wedge Vertebral Osteotomy For Treatment of Fixed Flexion Deformity of Spine in Ankylosis Spondilitis" by the authors Thiranont N. and Netrawichien P. Spine 1993, Dec.; 18 (16): p 2517-22, disclose that the average correction achieved has been of 33°. This technique may only be carried out in only one vertebral segment by region of the vertebral column. The different techniques of vertebral osteotomies have shown technical incovenients and complications derived from the surgical technique itself due to the bone resection and the necessary mobilization of the trunk and lower extremities during operations. These circumstances are broadly commented in an aticle by Ki-Tack Kim et al, Clinical Outcome Results of Pedicle Substraction Osteotomy in Ankylosing Spondylitis With Kyphotic Deformity. Spine. March 15, 2002, Vol. 17, number 6.

Another publication by Kostuik J.P. et al. Combined Single Stage Anterior and Posterior Osteotomy For Correction Of Iatrogenic Lumbar Kyphosis. Spine 1988 Mar, 13 (3) : p 257-66, refers to the need of resorting to two different interventions for the treatment of said deformities and the author's experience has achieved an average correction of 29°. Therefore, it is understood that the state in the art still requires important solutions in order to reduce the complications of vertebral osteotomies.

For the purpose of this review, the operative technique of an anterior opening wedge osteotomy is referred to as open wedge osteotomy (OWO). The technique of polysegmental lumbar posterior wedge osteotomies is referred to as polysegmental wedge osteotomies (PWO), and the monosegmental posterior closing wedge osteotomy is referred to as closing wedge osteotomy (CWO).

As result of the literature comparison made by the authors of the above-mentioned study, we may state the following:
These three techniques are in the field of major surgery. None of them could establish a predetermined correction angle. The three techniques require anterior or posterior plating such as Harrington 's rods and laminar hooks, or transpedicular screws.
a) The OWO technique needs disruption of the anterior longitudinal ligament. Patients with aortic calcification, poor medical risks, and patients with ankylosed hips (untreated with total hip arthroplasties) are contraindications for this operation; risks of lateral translation, vascular complications and mechanical risks (instability and pseudarthrosis in opening osteotomies) and loss of correction have been reported especially in OWO and PWO, whereas, minimal loss of correction occurred in CWO; permanent neurological complications have been reported in OWO
b) The PWO technique provides a more gradual correction without rupturing of the anterior longitudinal ligament. Full angular correction ofthe lumbar spine in PWO was not always achieved, thus resulting in a decreased correction or monosegmental correction. Loss of correction has been reported especially in OWO and PWO, whereas, a minimum loss of correction occurred in CWO. Permanent neurological complications have been reported in PWO. The PWO technique depends on the flexibility of the spinal discs, which are forced to expand perpendicularly.
c) The CWO technique minimizes the risk of stenosis with radicular compression or traction if an important correction is performed. The monosegmental correction in comparison with the OWO technique is faster and easier, with less bleeding than with the OWO technique. The reduction of the vertebral mass increases the risk of instability and infection. There is a shortening of the spinal length at least at the posterior region. A loss of correction has been reported especially in OWO and PWO, whereas a minimun loss of correction occurred in CWO. It may only be performed in only one vertebral segment by region of the vertebral spine and there is a detachment of the muscles that were fixed in the removed bone area. Likewise, there is a considerable continuous blood loss after the operation.

Their conclusion was "This structured review of the literature concerning three methods of lumbar osteotomy for correction of thoracolumbar kyphosis deformity showed that reports are limited and provide scant information on clinical data. Statistical analysis of the technical resulting data from these surgical methods was therefore not possible. Although the available data from the current literature suggest that CWO causes less serious complications and has better results, these data are not suitable for decision making with regard to which surgical treatment is preferable.

Furthermore, there is a need for a generally accepted clinical score that encompasses accurate measurements needed for preoperative and postoperative assessment of the spinal deformity in these patients."

### ii) Surgical methods for vertebral fusion, implants and intervertebral cages.

The number of spinal surgeries for the correction of the causes of low back pain has steadily increased over the last years. Most often, low back pain originates from damage or defects in the intervertebral disc between adjacent vertebrae. The disc can be herniated or may be suffering from a variety of degenerative conditions, so that in either case the anatomical function of the spinal disc is disrupted. The most frequent surgical treatment for these types of conditions consists in fusing the two vertebrae surrounding the affected disc. In most cases, the entire disc is removed, except for the annulus, by way of a discectomy procedure. Since the damaged disc material has been removed, something must be positioned within the intervertebral space, otherwise the space may collapse resulting in damage to the nerves extending along the spinal column.

In order to prevent said disc space collapse, the intervertebral space is filled with bone or a bone substitute in order to fuse the two adjacent vertebrae together. In early techniques, bone material was simply placed between the adjacent vertebrae, typically at the posterior zone of the vertebrae, and the spinal column was stabilized by means of a plate or fixation systems with rods that immobilized the affected vertebrae. By means of this technique, once fusion of vertebrae occurred, the material used to maintain the stability of the segment became superfluous. Moreover, the surgical procedures necessary to implant a rod or plate to stabilize the level during fusion were frequently lengthy and complicate.

It was therefore determined that a more optimum solution to achieve stabilization of an excised disc space consists in the fusion of the vertebrae between their respective end plates, most preferably without the need for anterior or posterior plating.

There have been numerous attempts to develop an acceptable intervertebral implant that could be used to replace a damaged disc and yet maintain the stability of the disc interspace between the adjacent vertebrae, at least until a complete arthrodesis is achieved. These "interbody fusion devices" have taken many forms. For example, one of the most frequent designs is a cylindrical implant. These type of implants are represented by the patents to Bagby, U.S. Patent No. 4,501,269; Brantigan, U.S. Patent No. 4,878,915; Ray, U.S. Patent Nos. 4,961,740 and 5,055,104; and Michelson, U.S. Patent No. 5,015,247. In these cylindrical implants, the outer portion of the cylinder can be threaded to facilitate insertion of the interbody fusion device, as depicted in Ray, Brantigan and Michelson's patents. Alternatively, some of the fusion implants are designed to be placed into the disc space between the vertebral plates. These types of devices are represented by the patents to Brantigan, U.S. Patent Nos. 4,743,256; 4,834,757 and 5,192,327.

In each of the above mentioned patents, the transverse cross section of the implant is constant throughout its length and is typically in the form of a right circular cylinder. Other implants have been developed for interbody fusion that do not have a constant cross section. For instance, the patent to McKenna, U.S. Pat. No. 4,714,469 shows a hemispherical implant with elongated protuberances that project into the vertebral end plate. The patent to Kuntz, U.S. Patent No. 4,714,469, shows a bullet-shaped prosthesis configured to optimize a friction fit between the prosthesis and the adjacent vertebral bodies. Finally, the implant of Bagby, U.S. Patent No. 4,936,848 is spherical and preferably positioned between the spinal center of the adjacent vertebrae.

Interbody fusion devices can be generally divided into two basic categories, namely solid implants and implants designed to permit bone ingrowth. Solid implants are represented by U.S. Patents Nos. 4,878,915; 4,743,256; 4,349,921 and 4,714,469. The remaining patents discussed above include some aspects enabling the bone to grow across the implant. It has been found that devices that promote natural bone ingrowth achieve a more rapid and stable arthrodesis. The device depicted in Michelson's patent is representative of this type of hollow implant which is typically filled with autologous bone prior to insertion into the intra-discal space. This implant includes a plurality of circular openings which communicate with the hollow interior of the implant, thereby providing a path for tissue growth between the vertebral end plates and the bone or bone substitute within the implant. In preparing the intra-discal space, the end plates are preferably reduced to bleeding bone to facilitate this tissue ingrowth. During fusion, the metal structure provided by Michelson's implant contributes to maintain the opening and stability of the motion segment to be fused. In addition, once arthrodesis occurs, the implant itself serves as a sort of anchor for the solid bone mass.

Another problem that is not addressed by the above prior devices concerns maintaining or restoring the normal anatomy of the fused spinal segment. Naturally, once the disc is removed, the normal lordotic or kyphotic curvature of the spine is eliminated. With the prior devices, the need to restore this curvature is neglected. For example, in one type of commercial device, the BAK device from SpineTech, as represented by the patent to Bagby, No. 4,501,269, the adjacent vertebral bodies are reamed with a cylindrical reamer that fits the particular implant. In some cases, the normal curvature is established prior to reaming and then the implant is inserted. This type of construction requieres a considerable penetration depth of the cylindrical implant into the generally healthy vertebrae adjacent to the instrumented discal space. However, this over-reaming of the posterior portion is generally not well accepted because of the removal of the load bearing bone of the vertebrae, and because it is typically difficult to ream through the posterior portion of the lower lumbar segment where the lordosis is greater. In most cases, with the use of implants of this type, no effort is made to restore the lordotic curvature, so that the cylindrical implant is likely to cause a kyphotic deformity as the vertebra settles around the implant. This phenomenon can often lead to revision surgeries because the spine becomes imbalanced.

The divergence angle between vertebrae that has been achieved by means of said procedures is very moderate, being in a range between 4° and 8°, as disclosed in a brochure from STRYKER Spine that has used Peek OIC material for spinal implants and Peek OIC cages in lordotic versions, available in 0°, 4° and 8° versions to be adapted to the lumbar lordosis.

Among the known intervertebral fusion methods wherein intervertebral devices are used there are US Patent No. 6,210,442B1 to Wing, referring to a cylindrical device which separates the vertebrae in a parallel way, US Patent No. 5,669,909 to Zdeblick and US Patent No. 5,683,463 to Godefroy, related to cylindrical-conical devices to be inserted between the vertebrae, which are separated with a little divergent angle. US Patents Nos. 5,443,514 to Steffee and 5,554,191 to Lahille relate to cuboid-sided devices, which once placed between the vertebrae, they separate them with a little angle between them. By way of example, said implants show that they may be used to separate vertebrae in a limited degree and consequently the correction achieved by the current devices that are inserted between the vertebrae is minimal and said implants cannot be used for important deformities in the curvature of the lumbar spine caused by the generalized disease of the intervertebral discs. It is bovious that these procedures are used to separate vertebrae but the curvature is not corrected in the usually necessary grades.

From a medical point of view, Bradley K. Wiener and Robert D. Fraser (Spine-Volume 23- Number 5 - 1998- Lippincon-Raven Publishers) have made an excellent classification of the interbody cage devices in the article "Spine Update - Lumbar Interbody Cages", which is included in this specification as a way of reference. We request to have this article included as part of the specification.

Intebody cage devices have been developed aiming to: (i) correct the existing mechanical deformation, (ii) provide stability to the segment until arthtodesis is obtained, (iii) provide the best possible enviroment for succesful arthrodesis, and (iv) achieve said aims with limited morbidity associated with the use of spinal cages.

Regarding mechanical deformation, an interbody cage device should restore the disc height, place annular fibers in a normal tension, create lordosis through the segment, reduce subluxed facet joints, obtain saggital balance through the segment, enlarge the neuroforaminal space, and restore to normal the proportion of weight borne through the anterior column.

With regard to mechanical stability, a interbody cage should provide immediate stiffness to the segment, be able to withstand the applied vertical loads, and provide adequate resistance to transferring and rotary forces in all directions.

Regarding optimal environment for arthrodesis, it should include a complete disectomy so that no intervening tissue lies between the bone fusion bed; a complete extirpation of the cartilaginous end-plate down to the healthy bleeding bone; preservation of the bone end-plate to maintain the structural integrity and discourage subsidence; use of the smallest cage size; use of implant techniques; provision of compression through distractive compression.

As far as the classification of such devices is concerned, Wiener and Fraser classify cages according to their structure (and according to the way said structure helps to achieve goals) and according to the materials (and the way said materials helps to achieve goals considering the biologic response, biomechanics and the possibility to show fusion by magnetic resonance or radiography).

In view of the present state in the art, there remains a need for a surgical method and an auxiliary device such as a cage and stabilizing devices, to achieve a spinal curvature correction above 10° and up to 70°, preferable about 29° and for a greater correction about 47°, that releases or reduces compression on the rachidian nerves at the foraminal area in those cases of discal degeneerative disease, that reduces the known adverse postoperative effects, that may pre-establish an angular correction to be achieved after the surgery, that provides the best possible environment for a successful arthrodesis and stability until arthrodesis is obtained, without disrupting the anterior longitudinal ligament, and forcing or removing the minimum number of discs, preferably only one disc. In comparison with the most accepted method for correcting the spinal curvature, that is to say the close wedge osteotomy technique, the method of this invention may be actually performed in two or more levels of the same spine region, since the intervention in the intervertebral disc is easier, and has a less bleeding for no fracture of the vertebral body is caused and only the intervertebral disc, which lacks blood vessels, is removed.

For the treatment of the disc degenerative disease, the surgical method developed herein and the device of this invention enable a great correction of the intervertebral angle similar to the one achieved by way of vertebral osteotomies but without resecting the vertebral body, without rupturing the anterior longitudinal ligament, without a major surgery and performing the procedure within the disc space between the adjacent vertebrae. This procedure carried out between the vertebrae, more precisely within the disc space, allows for the simultaneous operation in two contiguous or separate disc spaces in the same spine region, obtaining in this way a great correction of the spine curvature. Said correction cannot be reached by vertebral osteotomies since they may only be carried out in only one level by spine region.

The procedure of this invention, contrary to osteotomies, firstly expands the disc space by separating the vertebrae to their original state so that the spine is lengthened. This procedure opens the disc space from the front part when placing an anterior base wedge within said space. Therefore, it is a procedure that causes an addition without removing anything as the above-mentioned osteotomies.

A substantial difference with regard to the use of intersomatic implants (cages) is the way of releasing rachidian nerves. In "Spine, Orthopaedic Knowledge Update" by the American Academy of Orthopaedic Surgeons, 1997, Chapter 7: "Spondylosis: Degenerative Process of the Aging Spines", the participation of the subluxation of articular facets in the foramina shortening is described, wherein the penetration of the lower articular reduces the space for the normal emergence of the rachidian nerve due to the nearness of vertebrae secondary to the disc injury. Therefore, it is essential for the treatment of the disc degenerative disease to release said nerve.

As described herinabove in "Spine Update-Lumbar Interbody Cages", said implants and the method thereof release the rachidian nerve by separating the vertebrae and precisely aiming to enlarge the foraminal space and correct the articular facet subluxation, which is a necessary step for releasing the rachidian nerve roaming through the foraminal space. However, said separation of the vertebral bodies also separates the posterior edges of said vertebral bodies so that said implants cannot cause greater corrections of lordosis since they do not allow a convergence of the posterior area of the vertebral bodies.

The intersomatic method and implant herein disclosed resort to another solution different from the mechanical correction described in relation with cages in order to obtain an important lordosis correction. Said solution derives from the release of the rachidian nerve by means of an osteotomy, which removes the most important reduction element from the foramina when removing the top article of the lower vertebrae so that the subsequent approach of the posterior edges of the vertebral bodies does not interfere with the normal emergence of the rachidian nerve. Likewise, they provide enough space for the rachidian nerve passage upon closing the osteotomy for the approach of the posterior vertebral bodies, thereby determining a maximal correction of the angle between the vertebrae and space for the rachidian nerve as well.

In the same publication, it is stated that the purpose in the use of intersomatic implants is subluxation of the articular facets. Therefore, its preservation is obvious. This aspect of the surgical technique related to the preservation of the articular facets is in detriment to obtain a surgical field broad enough. This limitation has been noted in the literature about complications of the method. On the contrary, the osteotomy method proposed facilitates the exposure of the nervous tissue to the extent that said taught intervention enables the selection of the penetration level of the implant into the disc space thereby obtaining optional variants even during the operation as described in detail below.

The possibility of placing implants chosen from a set of different correction grades facilitates the formation of a harmonic curvature with different disc spaces corrected according to the needs of the particular case, as opposed to the closing wedge osteotomy, which causes an abrupt and acute break in the spine curvature for said kind of osteotomy is carried out in only one level and said aspect is not desirable since it is required that the spine has a harmonic curvature shape, regularly curved in all its length. The method enables a correction according to the deformity of the disc spaces in particular, for which there are a variety of different implant sizes to be adapted to the particular need.

The method requires the use of special intervertebral implants, which are the subject matter of this invention, and the addition of other different spine fixation apparatus.

After filing of our original application in Argentina, a new patent become available for which it deserves its study in regards to the present invention in order to avoid misunderstanding and to facilitate examiner analisys. We refer to Bryan's U.S. patent 6,500,206 B1 that discloses a spinal implant substantially rectangular shaped base section that includes a nose section and a method to use such implant. The present application describes and claim a trapezoidal actuangle shape for its implant. Bryan's patent says to achieve certain lordosis restoration by means of slots that are contoured to extend from the posterior tip about one third or more of the lenth toward the anterior tip of both vertebraes. The present invention achieves lordosis restoration by the angle of the trapezoidal shape. But the slots disclosed by Bryan's does not preserve the bone end-plate to maintain the structural integrity and discourage subsidence; moreover such cage is being supported by softbone within its critcial area. Of course Bryan's procedure should be forbidden for otheoporosis cases.

These slots should be contoured by the surgeon with no means to ensure depth nor angle for such slots, and above that, superior and inferior slots should be perfectly parellel to allow the cage insertion without further damaging the vertebrae. And the surgeon shall rely just upon his or her artisan's skills. This mehtod may not ensure a pre-determined lordosis correction that may vary upon the surgeon artisan's skills.

There is another important inconvenience about the use of slots, and comprises the way of handling rachidian nerves. To countour the slot corresponding to the superior vertebrae, the corresponding radichian nerve should be moved apart. It is well known in the state of the art and we have already metioned it above, that a substantial difference with regard to the use of intersomatic implants (cages) is the way of releasing rachidian nerves. Radichian nerve located within the border of the superior vertebrae is very difficult to be moved a certain distance without damage, and this fact is vital when choosing a cage and method of insertion. Since no special tecnhique is disclosed in this respect, we undertand that the method involves great risk of damage to the radichian nerve, considering the distance necessary to produce a slot and to handle a cutting instrument so close to the nerve. The method proposed in the present invention since is not acting on the posterior vertebrae face surface, does not need to move such radichian nerve.

Bryan's patent discloses serrated sides that are angled to prevent retraction as many other cages. But is we consider that Bryan's patent includes slots that are angled to form an angle open to the anterior section of the vertebrae, due to mechanical compression of the posterior portions of the vertabrae, forces are directed from the posterior portion to the anterior portion, and contrary to retraction, we should expect the cage to slide to the anterior section of the vertebrae. The present invention include serrated sides with a 'sea wave" shape that are angled to prevent to cage to slide to the anterior portion of the vertebrae, since retraction is voided by determined resection of the apophysis to fit and close completely the posterior section with mechanical compression.

The completely closing of the posterior opening in the present invention is not casual. Bryan's patent does not close the posterior opening and an open space like an 90 degree rotaded "8" is disclosed in pictures. This open sapce allows the invasion of healing tissue and provokes nerve adherence.

Descriptions of the invention made through out the above discussion should be consider as part of the present embodiment description and not just mere comments.

### SUMMARY OF THE INVENTION

In accordance with the present disclosure, the invention provides a new and improved spinal device and the surgical method thereof correcting spinal deformities. It is a method for causing an increase in the spine curvature by using a fish-shaped implant and said method consists in preparing the area by means of the osteotomy on the articular apophysis and spine apophysis of both vertebrae, following a skimming line over the area determined by the vertebra edge corresponding to the affected intervertebral space; a wedge osteotomy on the spine apophysis of the lower vertebra, wherein the angle determined by the cutting line of the spine apophysis of the lower vertebra and the cutting line of the spine apophysis of the top vertebra is an angle similar to the correction angle that will be obtained between the two vertebral bodies and corresponding to the angle formed by the upper and lower areas of the implant. After that, the disc is removed and the intervertebral space is prepared to receive the implant. The implant is placed with its vertex towards the posterior area of the vertebral spine and the rounded area towards the anterior area of the vertebral spine, without protruding from the perimeter of the vertebral bodies, said vertex completely positioned in the posterior edge of the intervertebral space allowing the existence of a free space in the anterior area of the intervertebral space; a compression force of the vertebral bodies is then caused on the upper and lower areas of the implant, which is used as a fulcrum for this step so that an open angle is formed towards the anterior area of the vertebral column, being said the angle formed bigger than that previously existing; osteosyntesis material is placed and finally the vertebrae are fixed with pedicular screws and osteosynthesis material is placed within the intervertebral space.

The implant is basically a trapezoid or acute-angled isosceles triangle where the area opposite the vertex or shortest base is a rounded surface, according to a vertical and perpendicular plan section view of the implant and it could be defined as an isosceles trapezoid which longest base corresponds to a semicircumference; a horizontal and perpendicular section to the implant sides will show a curved surface representing about a quarter of the circumference as a basically rectangular-shaped end (shortest side); the top and bottom surfaces include protuberances capable of penetrating into the vertebral mass through the vertebral plates. Said protuberances having a triangular shape are curved and bent (as a sea wave) in order to increase the implant stability after having been inserted. The protuberances extend from the beginning of the vertex to the maximum height of the implant gradually reducing their size.
The lateral view of the implant has a fish-like shape or water-drop shape and its rounded area would represent the fish head, the vertex protuberances would be the fish anal fin and the protuberances on the top and bottom areas would represent the fish fins. The implant comprises an inner hollow volume and holes on its surface which communicate with said hollow volume for the insertion of the osteosynthesis material and to enable and facilitate the osetosynthesis. The implant further includes a gap having a ring-shaped tunnel which hole is on the posterior surface or lateral surfaces to introduce the appropriate tools to be handled from a posterior or lateral access.

### BRIEF DESCRIPTION OF THE DRAWINGS

Various preferred embodiments are described herein with reference to the drawings wherein:
FIG A. Several vies of a modified version of the implant after filing the original priority application.
FIG 1. Side view scheme of the two adjacent vertebral bodies with normal intervertebral discs.
FIG 2. Side view scheme of two adjacent vertebral bodies with a collapsed intervertebral body.
FIG. 3. Side view of two vertebrae with a collapsed intervertebral disc and two osteotomy lines.
FIG. 4. Rear view of two contiguous vertebrae and the projection of the osteotomy lines.
FIG. 4A. A rear view of two contiguous vertebrae and the osteotomy of the upper vertebra.
FIG. 4B. Rear view of two contiguous vertebra and the osteotomy of the lower vertebra.
FIG. 5. Side view of two vertebrae wherein part of the intervertebral disc, the joints and part of the spine apophysis have been removed.
FIG. 6. Rear view of two contiguous vertebrae, wherein the joints and part of the spine apophysis have been removed.
FIG. 6A. Enlargement of the central region of Fig. 6.
FIG. 7. Side view of two contiguous vertebrae wherein an implant has been inserted between them together with two pedicular screws.
FIG. 8. Side view of one of the embodiments of the intervertebral implant herein described to be inserted between the vertebrae.
FIG. 9A: Top view of a vertebral body, wherein the final position of two implants inserted by means of a posterior surgery and the cutting line BB are shown.
FIG. 9B: Front view of two vertebral bodies, reconstruction along line BB wherein the relationship between two implants placed between both bodies is shown.
FIG. 10: Side view of two vertebrae with an implant between them wherein an approach of its rear part has been produced and where they contact the spine apophysis.
FIG. 11: Oblique view of the implant of Figure 8.
FIG. 12: Top view of the implant of Figure 8.
FIG 13: Oblique view of an implant to be inserted by a lateral surgery.
FIG. 14: Side view of an implant and the auxiliary tools for the insertion of said implant.
FIG. 15: Oblique view of an implant placed between two vertebrae and the corresponding tools.

### DESCRIPTION OF THE INVENTION

The surgical intervention herein proposed aims to decompress the rachidian nerves as trapped as consequence of the deformity of the degenerative disc disease and to correct the flattened spine deformity, which is also a consequence of the disc disease.

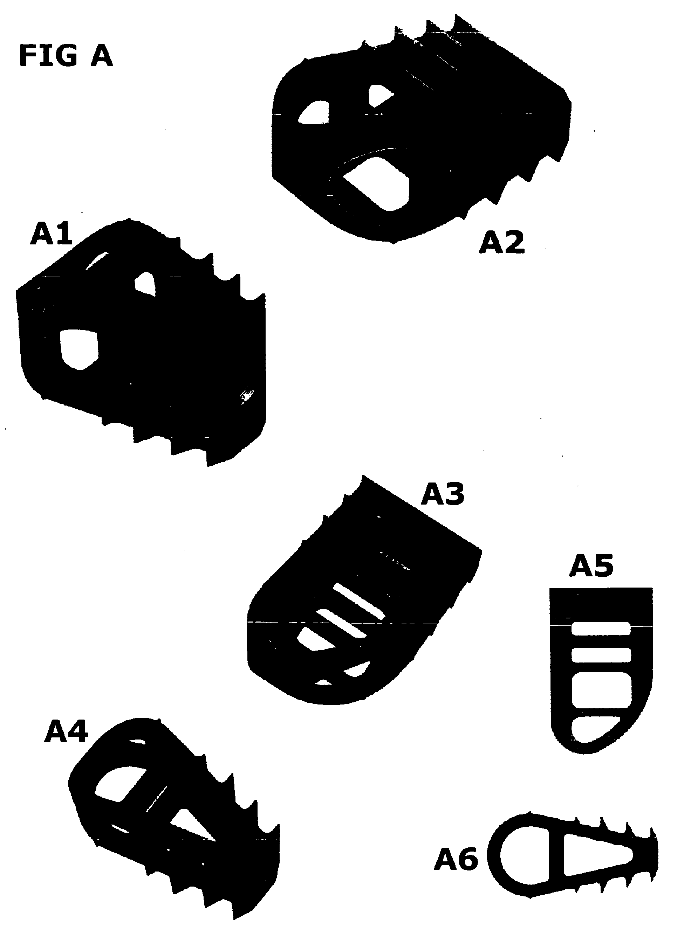

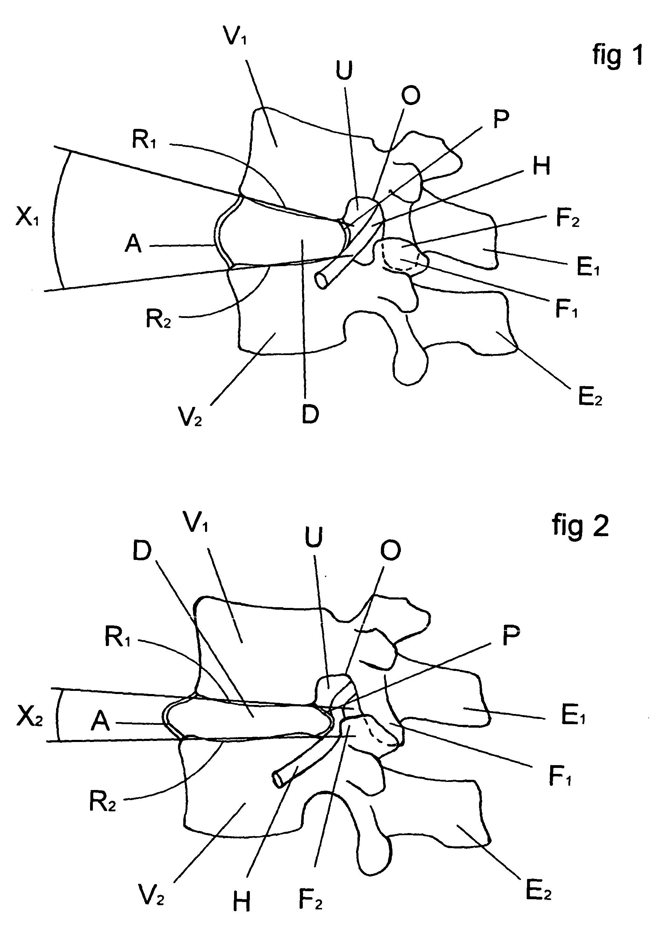

The natural state of a healthy disc is indicated in Figure 1, which shows a scheme of two adjacent vertebral bodies V1 and V2 and its healthy intervertebral disc D, for which both vertebrae V1 and V2 keep separate forming an open angle X1 forwards from two lines extending along their disc articular surfaces R1 and R2. Their anterior A and posterior P vertebral ligaments are tight. The rachidian nerve H emerges within the foraminal space U. The upper E1 and lower E2 spine apophysis are also shown.

In Figure 2, there is a scheme of two adjacent vertebrae V1 and V2 with its intervertebral disc D, depicting a disc degenerative disease and the approach of both vertebrae V1 and V2 and the subluxation of the articular apophysis F1 and F2. The subluxation of the articular facets modifies the size of the foramina U. The area named foramina U is a space defined by several anatomic elements. By the front side, there is the rear edge of the intervertebral disc D and the rear part of the upper vertebra V1, which continues backwards in a curve O ending in the upper articular apophysis F1, rear limit of the foramina U. Below the articular apophysis F2, it forms its lower limit. In view of said degenerative phenomenon, the lower articular apophysis F2 is projected penetrating into the foraminal space U. The same is done by the intervertebral disc D, which is enlarged and is projected into the foraminal space. In this way, the foraminal space U is reduced and the rachidian nerve H emerges from the foraminal tunnel U, trapped in its emergence spine place.

In the same Figure 2, a scheme is shown depicting two adjacent vertebral bodies V1 and V2 with the intervertebral disc D deteriorated, thereby causing the approach of their vertebral plates R1 and R2 and the laxity of the anterior A and posterior P vertebral ligaments. Said vertebral bodies have lost their normal relationship and the angle X2 open forwards has been reduced. In this way, the deterioration of several intervertebral discs, as noted in the vertebral degenerative disease, causes a stabilization of the normal lumbar spine curvature by the reduction of the angles between the vertebrae.

The osteotomy herein proposed produces a decompression of the rachidian nerve H by means of the resection of the articular apophysis F2 of the lower vertebra V2 and the resection of disc D, thereby enlarging the foramina U space and releasing the rachidian nerve H. Said nerve decompression is necessary to relief and eliminate the pain caused by the sustained compression of the nerve as a consequence of the deformity caused by the degenerative disc disease.

It is a further object of the osteotomy, as part of the surgical technique, to correct the flattening deformity of the vertebral column to recover the normal spine curvature. Therefore, it is necessary to remove part of the posterior bone elements by means of the osteotomy, otherwise the rear edges of the vertebral bodies would not been able to be approached due to the interference of said parts. The osteotomy comprises the resection of part of the so called neural arc. This is the posterior part of the vertebra, wherein more precisely the osteotomy comprises articular apophysis of the adjacent vertebrae and part of the spinal apophysis.

Once the osteotomy bones have been removed, the dura mater tube and the rachidian nerves may be seen. The rachidian nerves move towards the center enabling to see the intervertebral disc. The content of the intervertebral disc is emptied and an exhaustive cleaning of the soft disc and cartilaginous tissue is performed, with which the vertebral surfaces are prepared to receive the implant.

The first part of the correction is obtained with the expansion of the disc space by separating the vertebral bodies in the disc space. The second step of the technique consists in placing an implant which maintains separated only the anterior part of said vertebral bodies and the third step of this technique consists in approaching the posterior edges of the vertebral bodies and the edges of the osteotomy performed on the neural arc.

Simultaneously, the contact of the vertebral bodies occur. The closing of the osteotomy and the approach of the posterior vertebral bodies to each other is carried out by the action of different types of osteosynthesis materials known and frequent in the orthopaedic surgery, which are placed outside the vertebrae.

The use of stabilization systems generically named of pedicular screws is preferred. Said systems enable to place two screws outside the vertebrae, penetrating into them, each screw through each of the vertebrae sides, penetrating into the structure named pedicle. Said screws form a firm grasp in the vertebral body and on the one hand the screws of one side of the spine join together and on the other hand the screws of the other side of the spine join together. Said screws are joined to bars or plates in which they may be definitely fixed. Before fixation of the screws in the bars or plates, it is necessary to produce a compression force between the screws of the upper vertebra and those of the lower vertebra. This last action determines the closing of the osteotomy in the posterior part, the contact between the posterior vertebral edges and the compression of the implant placed between the vertebrae. In this way, the step of mechanical correction of the spine is finished.

### Preferred embodiment

Surgical intervention. In the surgical technique to be used with an intervention posterior to the spine, it is necessary to expose at least two adjacent vertebrae, which in their posterior aspect must remain exposed in a length no lesser than the distance between their transverse apophysis I and laterally up to the ends of the same transverse apophysis I.

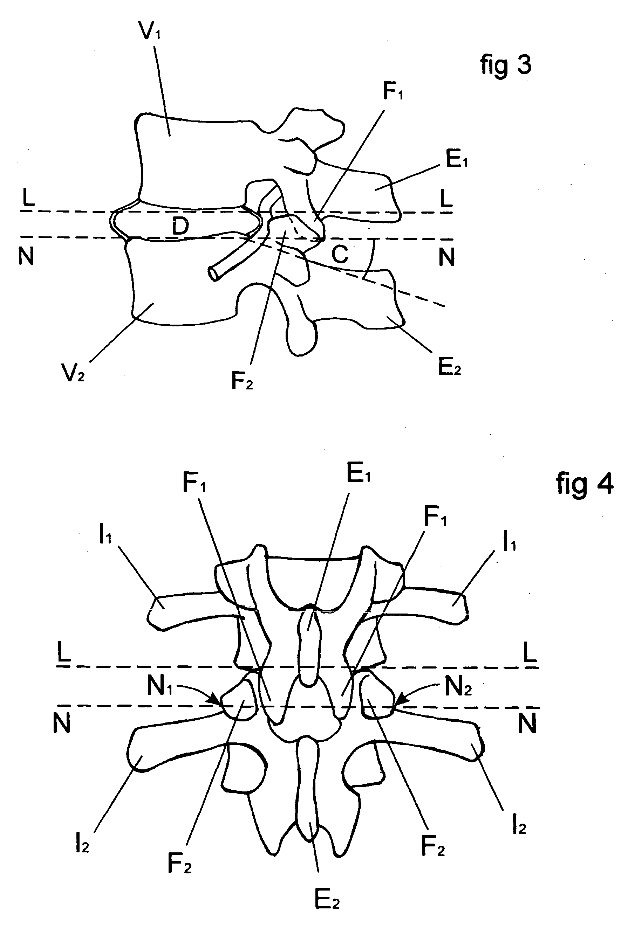

In Figure 3, the osteotomy of the upper vertebra V1 is performed on the articular apophysis F1 and the spine apophysis E1 if it is necessary according to the deformity of the latter, following the line L-L skimming the lower edge of the upper vertebra V1. The osteotomy of the lower vertebra is carried out on the lower articular apophysis F2 and the spine apophysis E2 following a line skimming the upper vertebral edge N-N of the lower vertebra V2.

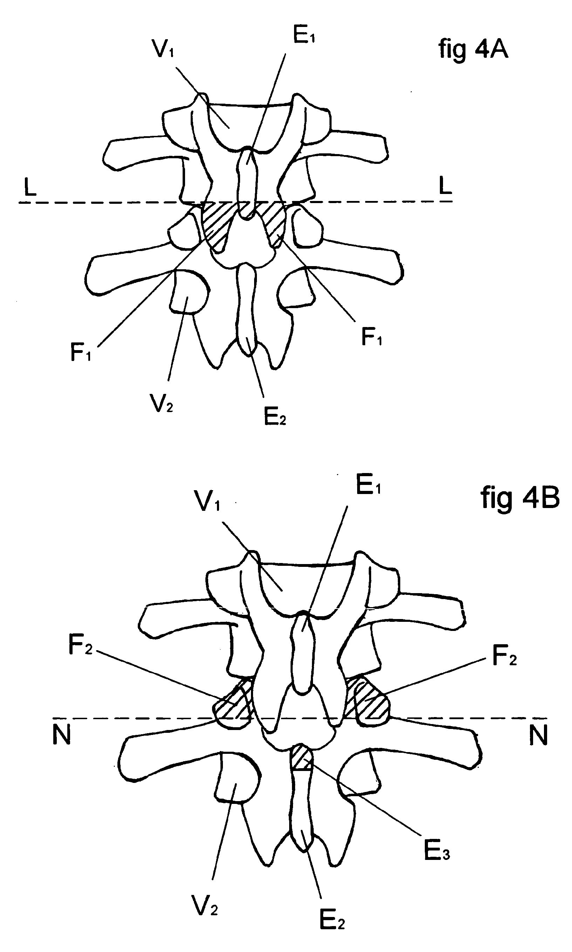

In Figure 4, the scheme view of the posterior part of the vertebrae shows the cutting line L-L of the upper vertebra V1 and the cutting line N-N of the lower vertebra V2. In view of the fact that the lines skimming the adjacent ends of the vertebral bodies L-L of the upper vertebra V1 and N-N of the lower vertebra V2, they cannot be seen during the intervention carried out through the posterior part of the vertebral column. The resort that simplifies the surgical technique consists in performing the osteotomy under the guide of anatomic elements easily identifiable in the surgically exposed field. In the vertebra V1, the line L-L coincides with the upper edges of the articular apophysis F2 of the lower vertebra V2. For the osetotomy of the lower vertebra V2, the skimming line N-N is taken as a limit to the points N1 and N2. Said points are easy to be anatomically defined since they are the union points of the upper part of the transverse apophysis I on the one hand and the lower articular apophysis F2. In this way, the upper vertebra V1 is removed, as shown in Figure 4A, the striped part of the articular apophysis F1 and of the lower vertebra V2, in Figure 4B, the striped part of the lower articular apophysis F2 and part o the spinal apophysis E3. In this way, once the osteotomy has been finished, the bone that has been cut is removed and when removing the upper articular apophysis F2 of the lower vertebra V2, the anatomical part penetrating into the foraminal space U and compressing the rachidian nerve H is removed. Then, with the removal of the intervertebral disc D, the decompression of the rachidian nerve H is completed as described in detail below.

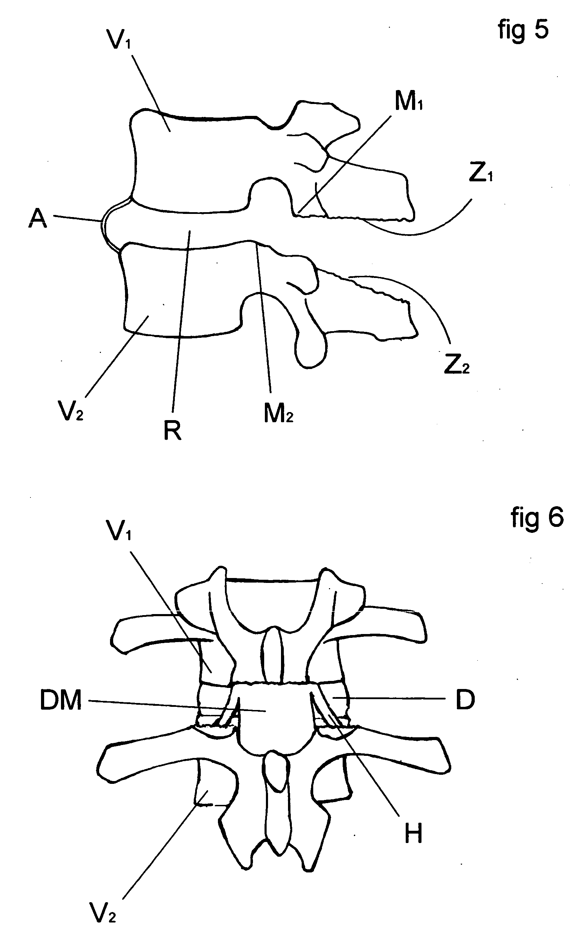

It is then necessary to make a second wedge-shape cut in the lateral aspect of the lower spinal apophysis E2, beginning in its end closest to the vertebral body wherein the vertex of said angle will be placed, as indicated in Figure 3. The degree of said angle C° between both cuts (cut of E1 on line L-L and the cut on the spinal apophysis E2) is similar to the correction angle that the vertebral body V1 and V2 will have. The result appears in the scheme of Figure 5, where M1 and M2 are the surfaces remaining after the osteotomies of the upper and lower articular apophysis F1 and F2; Z1 and Z 2 are the remaining surfaces of the spinal apophysis F1 and F2, and wherein R depicts the emptied intervertebral disc.

Preferably, the osteotomy angle in its lateral aspect will be of 29° at the beginning of the surgical intervention since it is the minimum correction achieved with the insertion of a smaller implant. If an implant for a greater correction degree is used during the intervention, said osteotomy must have the same degree as the implant to be used. In this way, when approaching the vertebral bodies V1 and V2 in their posterior part, there are no interferences by the removed vertebral bones. It should be pointed out that the osteotomy performed in this way always maintains a foraminal space equally broad for the rachidian nerve to pass through because the osteotomy angle is located behind the foramina and, consequently, whichever the required correction angle, for the range proposed, it does not significantly influence on the final size of the foramina and its relationship with the rachidian nerve.

Once the osteotomy of the posterior part of the vertebrae has been completed and the bone removed, the bone removed is kept for bone grafts necessary to be placed in the intervertebral space and in the posterior parts of the osteotomy.

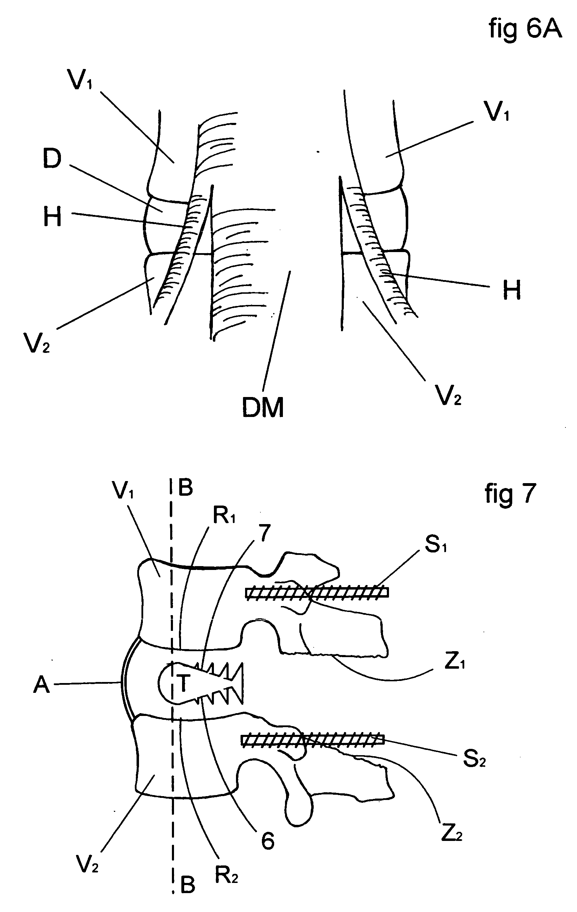

After removal of the osteotomy bones, the dura mater tube and the rachidian nerve may be directly seen, as indicated in Figure 6, wherein the rachidian nerves H emerge from the central dura mater tube DM. In Figure 6A, a detail of the relationship between the dura mater tube DM and the rachidian nerves H partially covering the intervertebral disc D is shown. Said rachidian nerves are moved towards the center, from one side at a time, so that the intervertebral disc D may be seen. After exposing the posterior part of the intervertebral disc D, a window is carved thereon by means of a variety of known tools and its content is emptied. The same steps are carried out in the opposite side. The following step consists in cleaning the vertebral end cartilage through the same windows carved in the intervertebral disc. After an exhaustive cleaning of the soft disc and cartilaginous tissue of the vertebral plates, different known tools are introduced by said windows to separate the adjacent vertebrae in a delicate and progressive way. Once the maximum separation has been achieved in accordance with the previous estimation, enough space between the vertebral bodies has been obtained to insert the predetermined implant.

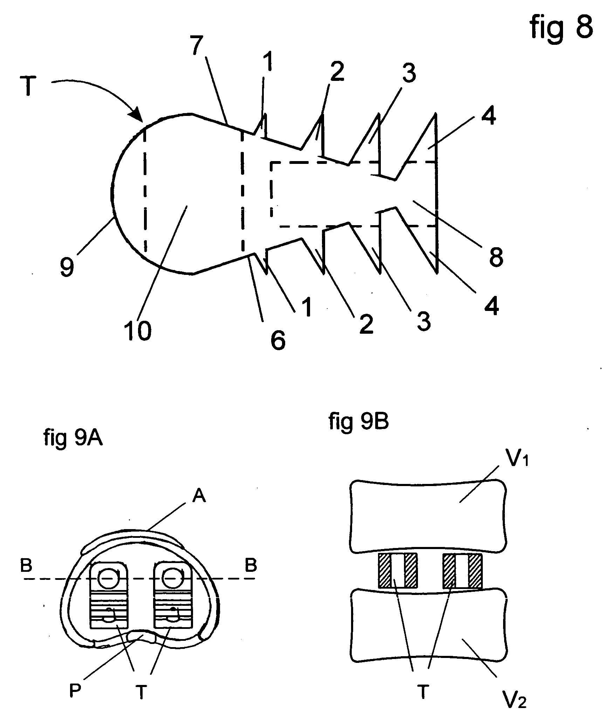

Implant T is taken by its posterior part with an accessory tool suitable for its handling, it is introduced through one of the carved windows until they are placed in the required position, as indicated in Figure 7 between the ends R1 of the upper vertebra V1 and the end R2 of the lower vertebra V2. Implant T may be held by means of a threaded instrument placed in opening 8 (See Figure 8) on the vertex face and it is placed with its face 9 forwards. In this way, implant T remains separating vertebrae V1 and V2 at the predetermined distance in the anterior region of the disc space. Between the vertebrae, the implant T remains initially as shown in Figure 7, with the angled supporting surfaces 6 and 7 of implant T free within the cavity. In this position of the implant T, it separates with its base both vertebral bodies in its anterior third part along line B-B. The accessory element is detached and the same steps are performed on the opposite side. That is to say that a second implant T is placed parallel to the first implant, thereby remaining both implants T inside the vertebral edges without rpotruding as shown in Figure 9A (Top view) and 9B (line cut B-B).

After having placed the implants, bone grafts are placed between said implants filling the free places of the intervertebral disc.

Placement of other external means of vertebral fixation. Pedicular screws S1 and S2 of common use un the art of vertebrae fixation are placed in both vertebrae V1 and V2 respectively, as shown in Figure 7.

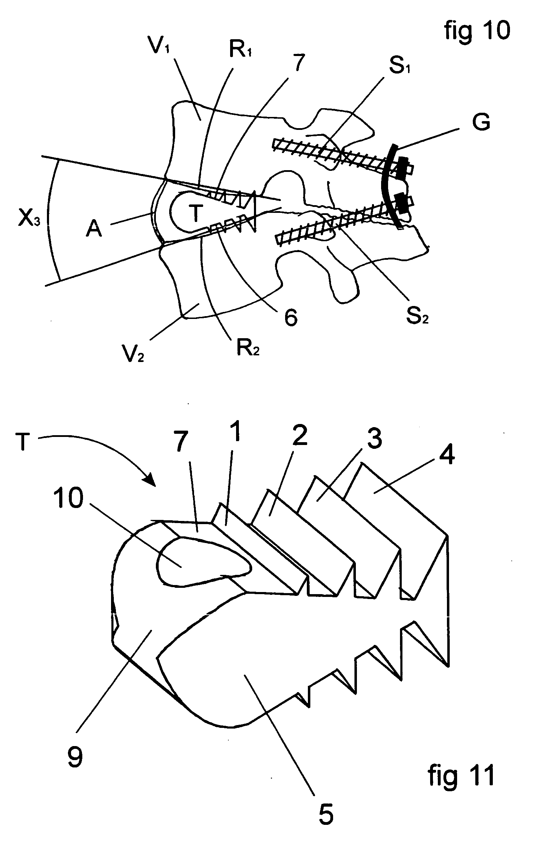

In Figure 10, there is a scheme of the way in which by the concentric pressure exerted on the pedicular screws 1 and S2 of the upper and lower vertebrae V1 and V2, the closing of the disc space is obtained in its posterior part due to the fact that the implant T keeps the vertebrae separated in their anterior parts. Said closing between vertebrae V1 and V2 determines the support of surfaces R1 and R2 on surfaces 6 and 7 of implant T, wherein the upper fins penetrate into a vertebral surface R1 and the lower fins penetrate into another vertebral surface R2. In this way, when the vertebrae approach in their posterior parts, they also contact the bone surfaces Z1 and Z2 and the implants T are trapped between vertebrae V1 and V2. The implants T hold vertebrae V1 and V2 and both vertebral plates form an angle X3 determined by the triangular shape of the implant T. The screws S1 and S2 are fixed in the bars or plates of common use G, and the assembly is immobilized with nuts.

This action determines the closing of the intervertebral space only in its posterior part, since the implants prevent the anterior edges of the vertebral bodies from approaching. The closing of the posterior osteotomy occurs simultaneously with the contact of the posterior edges of the vertebral bodies. In this way, the implants serving as support between the vertebral bodies, adapting their sides to the ends of the vertebrae, are trapped.

This continuity between vertebrae and implants create the support necessary for the body weight load. Likewise, in view of their triangular shape and the posterior closing of the vertebral bodies, these implants avoid their migration to the posterior area. Moreover, the presence of fins in the implant sides offer another anchor means of the implant when penetrating into the vertebral bone.

The intervention is finished with the placement of bone grafts in the union area of the osteotomy in order to promote fusion. Bone grafts may be used to be placed in the disc space R between implants T, immediately after the placement thereof or in the external parts of the spine in a known way as well as within the hollow volume of the implant.

### Intervertebral implant

### (<New text)

The implant described below has certain differences over the implant described in the priority document, owing to the various constructive tests carried out in order to achieve the final product.
The above-mentioned notwithstanding, said differences "may be considered as described and taught" in the priority document.
A new set of drawings, Fig. A, is added to exemplify the implant after having worked over it. The improved implant of this invention is illustrated in Figures A1 to A6, which describe an implant having a "fish-like" shape or a "curved water-drop" shape. A lateral view of the implant is represented in Figure A6 showing an isosceles trapezoid which longer base is a semicircumference.
A plan view of the implant is represented in Figure A5 showing a rectangle that has been "flattened and curved in the vertex of a shorter side with a longer side and curved without flattening in the opposite vertex of the same shorter side. Figure A2 showing a front-lateral view of the implant and Figure A3 of the implant are useful to show the front or head of the implant: a pyramidal form of walls that are curved and off-center towards a side of the implant. The off-center location is useful not to harm the surfaces of the vertebral plates, since in that area the structure of the discal cavity imitates the shape of biconvex lens.
The improved implant, which is basically a hollow body, includes a different array and has more holes to increase the amount of osteosynthesis material to be placed between the vertebral plates. Its vertex includes a ring-shaped tunnel as a means to place supporting tools for the insertion of the implant.
The "fins" or protuberances or teeth in the top and bottom faces have been slightly modified. Now, its shape is not just a right-angled triangle but said triangle has been curved and pointed in a way similar to a "sea-wave" thus maximizing the stability of the device since the slipping resistance has been increased towards the pushing forces caused by the vertebral plates against the top and bottom faces. The proportions of the implant have also been modified according to the vertex, which no longer has the characteristic "fish anal fin-like shape" but it includes its corresponding pointed protuberances.

The top and bottom faces containing the protuberances are the faces that will remain in contact with the vertebral plates. The angle between said faces determines the intended angular correction achieved by means of the insertion of said implant.

With regard to the angular correction, the smaller implant has a 8 mm base, with which an angular correction of 18° is achieved. Said angle size is suitable to be used in the upper areas of the lumbar zone.

### (End of new text>)

### Preferred embodiment as desribed in the original priority application

The intervertebral implant of this invention is illustrated in Figures 8 to 11. It has a triangular configuration if a cross section is made tending to an isosceles triangle, which two bigger sides 6 and 7 are supported on the vertebral surfaces. The vertex ends with two triangular projections 4, one of the is the upper one and the other, the lower one, with an orientation almost perpendicular to the vertebral surfaces. A more exact description may state that a lateral cross section would show a fish-shape with several upper and lower fins in its body and an anal fin comprising two aligned and opposite fins. A lateral or posterior cut will have a rectangular shape as well as a top or bottom view.

The longest sides determine the correction angle that will be obtained with the implant insertion between the two vertebrae.

In the region opposite the vertex, the implant includes one or two big holes through which the bone material or an equivalent material thereof is inserted to favor the bone fusion. The protuberances on the upper and lower surfaces ("fins") serve as a fixation means of the implant to the vertebral plates, thereby acting as a fixation means, to avoid the displacement of the implant.

The material with which the implant is constructed may be any material suitable for its purpose, as described and analyzed in the prior art, such as metal, different alloys and compounds available in the market.

The implants may be 20mm long and they may have a 10 mm minimum height in their base to obtain a 20° correction. If they had a 15mm base, there would be a 47° correction. A variety of implants will be available to apply according to the desired correction.

Additionally, the fins may be saw-shaped for a better fixation to the vertebral surface and the implant body may be hollowed through its anterior and posterior ends and it may also include holes in the upper and lower surfaces in order to increase the volume of bone material and its communication so that the vertebral fusion be favored.

Likewise, a threaded tunnel is provided in the lateral, anterior or posterior part of the implant a means for placing the supporting instrument to place the implant in the intervertebral space. Then, said tunnels may act as holes enabling to increase the volume of bone material and its communication with the above-commented beneficial effects.

Figure 11 is an oblique view of the implant. Its length is not greater than 20 mm and its minimum height is of 10 mm. It has a rounded anterior base 9 and its body 5 ends in a posterior vertex. On the supporting side 6, there are fins 1, 2 and 3 and on the supporting side 7 there are a set of fins 11, 12 and 13, opposite to the ones of the supporting side 6. A threaded tunnel 8 begins in a hole in the posterior part of implant T as may be seen in Figure 8.

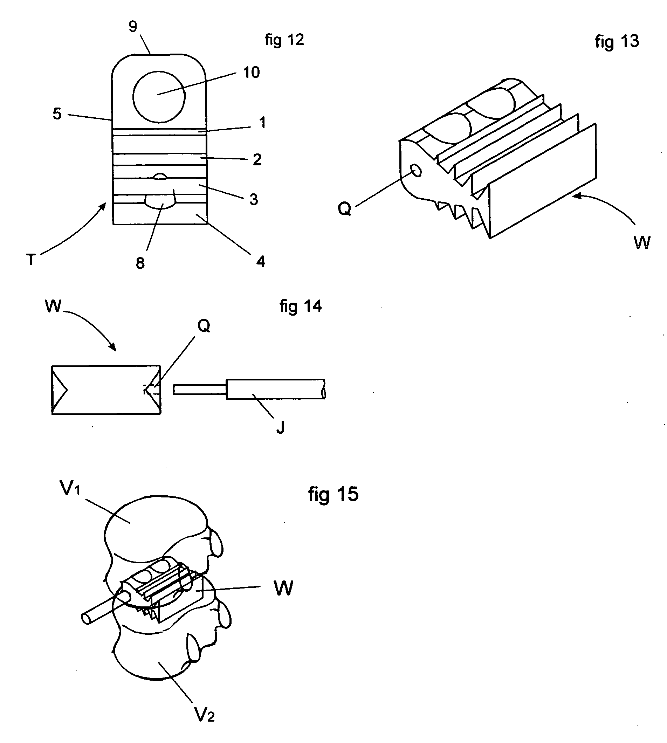

Figure 12 is a top view of the mentioned implant.

Figures 13 and 14 show implant W being a variant of implant T, which is the suitable implant to be inserted by lateral surgery. Its side view is identical to that of implant T and its width has a maximum of 30mm. It also has a threaded hole Q for the placement of an instrument J of Figure 14.

### Other embodiments of the present invention

Among the possible variants of the method of this invention, it must be considered that only one implant may be used to achieve the same correction effect, using the remaining disc space for the insertion of a considerable amount of bone grafts. The benefit of this variant would consist in a greater amount of bone mass for a rapid fusion between the vertebral bodies.

Another embodiment of this invention consists in placing the implant T using the same posterior intervention, through an opening in the more lateral space disc. Consequently, a lesser separation of nervous tissue is required. This embodiment is possible because the previous osteotomy provides enough space to choose the most convenient insertion path of the implant T according to the surgical habit or to the particular case. The insertion path of implants in the intervertebral space may consists either in following directly an orientation from the back forwards by the posterior part of the disc space or by a more lateral path penetrating from a more lateral point, thus significantly avoiding the separation of the nervous tissue. This must be taken into consideration in cases of reoperation, wherein the dissection of the rachidian nerves for their separation is difficult due to the previous cicatrization thereon, where the possibilities of complications related to said cause increase, or if any accidental injury occurs during the surgery thus making it difficult to separate said rachidian nerves. In this cases, the possibility of selection during the surgical intervention of the placement path of the implant is significantly useful. The surgeon may then choose the placement of two implants T as above-described or the placement of an implant T using the same path, penetrating into the posterior intervertebral space from the right or the left side, or using the same surgical intervention path to place two implants T penetrating into the intervertebral space more laterally, or with the same lateral penetration point, the surgeon may choose between placing one implant from the right side or from the left side. In this way, the surgeon will have six options of the technique application, with the placement of the same implant T to achieve an identical correction.

Another embodiment is the insertion of the implant by a lateral surgical path. This variant requires a surgical intervention from behind the peritoneum, that is to say without penetrating into the abdominal cavity. The placement of the implant requires the extirpation of the intervertebral disc and the cartilage of the vertebrae ends. The same steps are followed to expand the space between the vertebral bodies and the implant W is placed in the suitable position with an instrument holding the implant by its lateral aspect (Fig. 15). Once the implant has been positioned separating the vertebrae by its anterior part, the following step continues with an osteotomy in the posterior part of the vertebrae, to be carried out through a posterior intervention path. In this case, it is possible to make two interventions simultaneously, the lateral and the posterior ones, in which case the osteotomy, the placement of the pedicular screws and the closing thereof adjusting the pedicular screw system is performed in the posterior intervention.

Although this embodiment implies two surgical interventions, the first one through a lateral path behind the peritoneum and the second one through the posterior part of the spine, it has numerous advantages in the cases where correction is necessary in the high lumbar disc spaces, wherein the presence of the medulla makes it impossible to carry out the intervention of the disc space through a posterior path since it would be necessary to move the medulla, which implies serious consequences.

Therefore, in those levels of the vertebral column, no operations can be made on the intervertebral disc through a posterior path and, consequently, it is preferred to act when necessary through other paths. The mentioned variant enables the angle correction by placing an implant W through a lateral path and an osteotomy through the posterior path, so that it is not necessary to handle the medulla for the proposed intervention. This variant should be considered for those cases which need corrections in regions such as the thoracolumbar region. It is a region with considerably frequent residual deformities, fracture sequels, owing to the fact that it is a region where there exists a transition between a region having greater stability, such as the thoracic, for the presence of the thoracic cavity and the lumbar region having a lesser mechanical stability. The second reason is the transition area between the lumbar lordosis curvature, which is concave, continuing with the dorsal kyphosis, which is convex. A vertebral injury causing a vertebral wedging in that area produces a very significant distortion in the lateral alignment of the spine, thereby causing an unbalancing sequel for bipedestation and pain. Most methods resort to other spine regions to solve this problem by means of osteotomies in other levels where there is no medulla, for example the low lumbar region, but at the expense of violating a healthy region. The method herein proposed may be used in the same level where there exists a deformity and it also enables restoration of the harmed spine harmony, without removing the harmed vertebrae and only using intervertebral spaces.

Moreover, this method may be completed by placing other means for the mechanical fusion between vertebrae using the lateral retro peritoneal path, such as the screws inserted in each of the adjacent vertebrae joined by means of bars.

## Claims

1. An implant to be inserted in the disc space between two adjacent vertebrae for the correction of the vertebral spine curvature **characterized in that** its configuration (lateral view) is basically a wedge or acute-angled isosceles trapeze, wherein the area opposite the shorter base or opposite to the vertex is a rounded pyramid-like surface, and the upper and lower surfaces of the trapeze include fixation means to the vertebral plates of the adjacent vertebrae.

2. The implant of claim 1, **characterized in that** the area opposite the vertex that is a rounded pyramid-like surface is not centered, but laterally shifted to one side.

3. The implant of claim 1, **characterized in that** the fixation means are protuberances capable of penetrating into the vertebral mass through the surface of the vertebral plates, and such protuberances are saw-shaped (curved) extended along the width of the upper and lower surfaces and oriented to prevent horizontal slipping in the direction of the anterior portion of the vertebrae.

4. The implant of claim 1, **characterized in that** may include a hollow volume inside it and holes in its surfaces, being said holes communicated with said hollow volume for the insertion of osteosynthesis material and to enable and facilitate the osteosynthesis.

5. The implant of claim 1 **characterized in that** may inlcude:
a) the area opposite the vertex that as a rounded pyramid-like surface laterally shifted to one side;
b) fixation means as protuberances capable of penetrating into the vertebral mass through the surface of the vertebral plates, and such protuberances being saw-shaped (curved) extended along the width of the upper and lower surfaces and oriented to prevent horizontal slipping in the direction of the anterior portion of the vertebrae;
c) a hollow volume inside it and holes in its surfaces, being said holes communicated with said hollow volume for the insertion of osteosynthesis material and to enable and facilitate the osteosynthesis.

6. The implant of claim 5, **characterized in that** it includes a hollow cavity in a threaded tunnel shape, which opening is in the posterior surface of the implant, for the insertion of tools suitable for handling through a posterior access.

7. The implant of claim 5, **characterized in that** it includes a hollow cavity having a threaded tunnel shape, which hole is in any of the two lateral surfaces, and in the area opposite the vertex, for the insertion of tools suitable for handling through a lateral access.

8. A method for producing an increase in the vertebral spine curvature by using the implant of claim 1, **characterized by** the following steps:
a. releasing the access and handling area to a determined intervertebral space;
b. preparing the intervertebral space to receive the implant;
c. placing the implant with its vertex towards the posterior area of the vertebral column, and the rounded area towards the anterior area of the vertebral column, without protruding from the perimeter of the vertebral bodies and said vertex being completely positioned in the posterior end of the intervertebral space and enabling the existence of free space in the anterior area of the intervertebral space;
d. exerting a posterior compression force of the vertebral bodies over the upper and lower surfaces of the implant, using said implant as fulcrum for this operation, so that an angle open towards the anterior area of the vertebral column is formed and said angle being bigger than the previously existing one.
e. placing the osteosynhtesis material;
f. fixing the vertebrae by means of external fixation means.
g. placing the osteosynthesis material in the intervertebral space.

9. The method of claim 8, **characterized in that** the compression force is exerted by means of the pressure exerted over pedicular screws placed on the vertebrae forming the intervertebral space.

10. The method of claim 9, **characterized in that** pedicular screws are used as an external fixation means, fixing said screws to bars or plates and immobilizing said assembly with nuts or other fixation means.

11. The method of claim 8, **characterized in that** the release of the access and operation area to the intervertebral space consists in the osteotomy of the articular apophysis and the spine apophysis of both vertebrae, following a line skimming the surface determined by the vertebra edge corresponding to the affected intervertebral space, an wedge-shaped osteotomy on the spine apophysis of the lower vertebra, wherein the angle determined by the cutting line of the spine apophysis of the lower vertebra and the cutting line of the spine apophysis of the upper vertebra, is an angle similar to the correction angle that will be obtained between the two vertebral bodies and corresponding to the angle formed by the upper and lower surfaces of the implant.

12. The method of claim 8, **characterized in that** the preparation of the intervertebral space to receive the implant consists in moving the dura mater tubes and the rachidian nerve to one side, thereby exposing part of the intervertebral disc; carving a window in the disc, and emptying the disc with suitable instruments; performing the same two steps in the opposite side; cleaning the soft and cartilaginous disc tissues of the vertebral plates through the carved windows; separating the adjacent vertebrae in a delicate and progressive way with appropriate instruments.

13. The implant of claim 1, **characterized in** the angle formed by the upper and lower surfaces is of at least 10 degrees, preferably between 18 and 70 degrees, more preferably between 18 and 47 degrees, most preferably 18 degrees, 29 degrees and 47 degrees..

14. The implant of claim 13, **characterized in that** its length is not greater than 20 mm and its minimum height is of 8 mm and its maximum height is of 30 mm depending on the angle to be achieved and size of the vertebrae.

15. The implant of claim 5, **characterized in that** it is made of metal, titanium alloy, biocompatible material, or any other material suitable for said purpose.
